Europäisches Patentamt

European Patent Office

Office européen des brevets

(11) Veröffentlichungsnummer: **0 392 031**
**A1**

## (12) EUROPÄISCHE PATENTANMELDUNG

(21) Anmeldenummer: **89106272.1**

(22) Anmeldetag: **10.04.89**

(51) Int. Cl.⁵: **A61N 5/10**

(43) Veröffentlichungstag der Anmeldung:
**17.10.90 Patentblatt 90/42**

(84) Benannte Vertragsstaaten:
**CH DE FR GB LI SE**

(71) Anmelder: **Siemens Aktiengesellschaft**
**Wittelsbacherplatz 2**
**D-8000 München 2(DE)**

(72) Erfinder: **Schmidt, Ernst-Ludwig, Dr. rer. nat.**
**Brunnenstrasse 57**
**D-6750 Kaiserslautern-Dansenberg(DE)**

(54) **Strahlentherapiegerät mit bewegbarer Blende.**

(57) Die Erfindung betrifft ein Strahlentherapiegerät, bei dem der Isodosenverlauf sowohl durch eine während der Bestrahlung gesteuert bewegbare Blende als auch durch einen nicht beweglichen Filterkörper im Strahlengang beeinflußbar ist. Dabei ist der Filterkörper (15) so angeordnet, daß er eine in Öffnungsrichtung der Blende (12a) abnehmende Absorption aufweist. Dagegen bewirkt die Blende (12) eine in ihrer Öffnungsrichtung abfallende Dosisleistung. Durch Überlagerung beider Effekte wird es möglich, den Isodosenverlauf im Bestrahlungsobjekt in Öffnungsrichtung abfallen oder ansteigen zu lassen, so daß man eine große Variationsbreite möglicher Isodosenverläufe erhält, ohne den festen Filterkörper (15) wechseln zu müssen.

FIG 1

EP 0 392 031 A1

## Strahlentherapiegerät mit bewegbarer Blende

Die Erfindung betrifft ein Strahlentherapiegerät mit einer Strahlenquelle und einer zwischen Strahlenquelle und Bestrahlungsobjekt angeordneten, ein Bestrahlungsfeld bestimmenden Blendenanordnung, wobei während der Bestrahlung mindestens eine Blende der Blendenanordnung durch eine Steuereinrichtung so bewegbar ist, daß eine in Öffnungsrichtung der Blende abfallende Dosisleistung im Bestrahlungsfeld erzielbar ist.

Eine derartige Anordnung ist beispielsweise aus dem Artikel "Wedge-Shaped Dose Distributions by Computer-Controlled Collimator Motion" in Medical Physics (5), Sept./Okt. 1978, Seiten 426 bis 429, bekannt. Dabei wird während der Bestrahlung durch eine definierte Blendenbewegung eine keilförmige Isodosenform erzielt, wie sie häufig in der Strahlentherapie zur Anpassung an die anatomischen Gegebenheiten des Behandlungsobjektes gewünscht wird. Die keilförmige Isodosenform beruht darauf, daß unterschiedliche Bereiche des Bestrahlungsfeldes der Strahlung verschieden lang ausgesetzt sind. Die notwendige Bewegung der Blende wird durch ein iteratives Verfahren ermittelt.

Die bewegliche Blende kann als Ersatz für sonst übliche keilförmige Filter angesehen werden. Durch Anbringung eines keilförmigen Filters zwischen Strahlenquelle und Bestrahlungsobjekt kann man ebenfalls einen keilförmigen Isodosenverlauf erreichen; jedoch muß hier in Abhängigkeit vom gewünschten Isodosenverlauf jeweils das Filter gewechselt werden.

Hingegen ist es bei der beweglichen Blende nachteilig, daß die Dosis stets in einer festgelegten Richtung, nämlich entgegengesetzt zur Öffnungsrichtung der Blende ansteigt.

Aufgabe der Erfindung ist es, ein Strahlentherapiegerät der eingangs genannten Art so zu verbessern, daß eine größere Flexibilität des einzustellenden Isodosenverlaufes möglich wird.

Diese Aufgabe wird erfindungsgemäß dadurch gelöst, daß zusätzlich ein nicht beweglicher Filterkörper in den Strahlengang einfügbar ist, der eine in Öffnungsrichtung der Blende abnehmende Absorption aufweist. Damit kann ein Isodosenverlauf erzielt werden, der im Untersuchungsbereich z.B. ansteigt und dann wieder abfällt. Im Gegensatz zu Anordnungen, die lediglich einen austauschbaren Filterkörper und keine Blende mit einer Bewegungssteuerung aufweisen, kann man hierbei mit einem einzigen Filterkörper eine große Variation an Isodosenverläufen erzielen.

Vorteilhafte Ausgestaltungen der Erfindung sind in den Unteransprüchen angegeben.

Ein Ausführungsführungsbeispiel der Erfindung wird nachfolgend anhand der Fig. 1 bis 3 näher erläutert. Dabei zeigt:

Fig. 1 den schematischen Aufbau eines Strahlentherapiegerätes mit einem Elektronenbeschleuniger,

Fig. 2 verschiedene mit der erfindungsgemäßen Anordnung erzielbare Isodosenkurven, und

Fig. 3 eine schematische Anordnung von Blende, Filterkörper und Körper des Behandlungsobjektes.

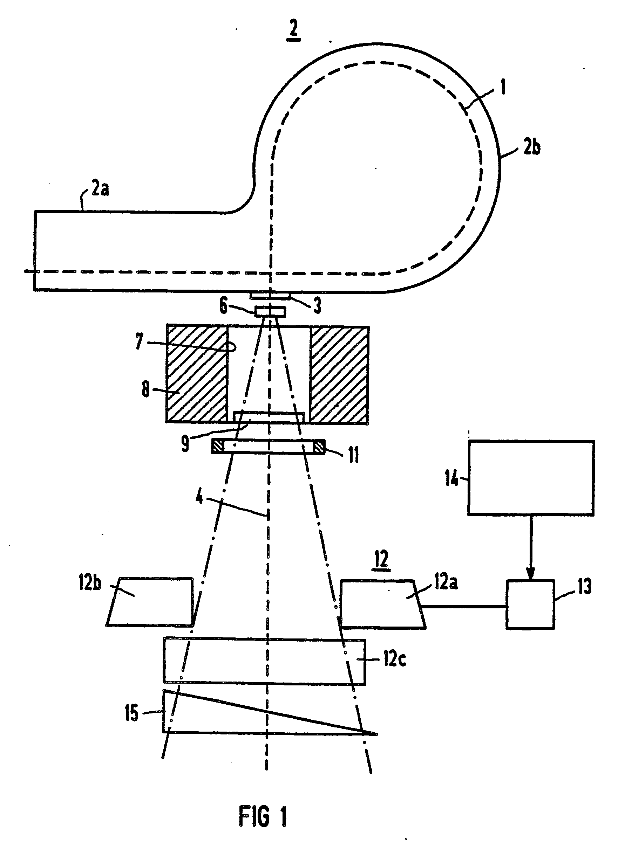

Der prinzipielle Aufbau eines Strahlentherapiegerätes mit einem Elektronenbeschleuniger ist in Fig. 1 schematisch dargestellt. Ein in einem Elektronenbeschleuniger 2a erzeugter Elektronenstrahl 1 wird durch einen Umlenkmagneten 2b auf eine Kreisbahn gelenkt und durch ein Fenster 3 auf eine Achse 4 ausgerichtet. Der Elektronenstrahl trifft dann auf eine erste Streufolie 6, passiert eine Öffnung 7 eines Abschirmblockes 8 und trifft auf eine zweite Streufolie 9. Anschließend wird er durch eine Meßkammer 11 geschickt, mit der die Dosisleistung erfaßt wird. Wenn man die erste Streufolie 6 durch ein Target ersetzt, kann man die Elektronenstrahlung auch in eine Röntgenstrahlung umwandeln. Schließlich ist im Strahlengang eine Blendenanordnung 12 vorgesehen, mit der das bestrahlte Feld des Untersuchungsobjektes festgelegt wird. Die Blendenanordnung 12 besteht aus vier einzelnen Blenden 12a bis 12d, wobei die Blende 12d in Fig. 1 nicht sichtbar ist, weil sie durch die Blende 12c verdeckt ist. Die Blenden 12a bis 12d geben ein rechteckförmiges Feld frei.

Die einzelnen Blenden können von Hand oder motorisch verstellt werden. In Fig. 1 ist dies bezüglich der Blende 12a durch eine Antriebseinheit 13 angedeutet, die von einer Steuereinheit 14 gesteuert wird. Für die nachfolgend beschriebene Methode zur Bestimmung der örtlichen Dosisverteilung ist die motorische Verstellbarkeit wenigstens einer der Blenden 12a bis 12d Voraussetzung.

Unterhalb der Blendenanordnung 12 ist schließlich noch ein keilförmiges Filter 15 vorgesehen.

Der Aufbau eines Strahlentherapiegerätes mit einem Elektronenbeschleuniger ist in der US-PS 4 121 109 näher erläutert.

Wie bereits eingangs erwähnt, ist bei der Strahlentherapie häufig ein keilförmiger, d.h. unter einem bestimmten Winkel verlaufender Isodosenverlauf erwünscht, wobei bisher dieser Verlauf durch ein keilförmiges Filter erzielt wurde. Wie ebenfalls bereits eingangs erwähnt, gibt es jedoch bereits auch Vorschläge, den Isodosenverlauf durch eine bewegte Blende zu beeinflussen. Bei den bekannten Vorschlägen wird die Bewegung der Blende dabei iterativ ermittelt. Die notwendige Blendenbewegung der Blende 12a für einen

Winkel $\alpha$ der Isodosenkurve läßt sich aber nach folgender Gleichung auch analytisch vorgeben:

$$v(x) = \frac{k}{I_0} \frac{\exp(x-B) \ tg \, \alpha}{\mu \ tg \, \alpha}$$

Dabei ist $v(x)$ die Geschwindigkeit der Blende 12a, $I_0$ und K die Dosis bzw. Dosisleistung in Luft bei der Tiefe O, B die Feldbreite, x die Blendenposition, $\mu$ der effektive lineare Schwächungskoeffizient und $\alpha$ der gewünschte Isodosenwinkel. Dieser analytische Ansatz ist nur deshalb möglich, weil hierbei keine Streueffekte der Teilfelder, d.h. der Felder in den einzelnen Blendenpositionen, berücksichtigt werden.

Überraschenderweise hat sich jedoch herausgestellt, daß dies nur zu vernachlässigbaren Fehlern führt. Dies rührt von der Tatsache her, daß das vollständig geöffnete Feld zeitlich wesentlich höher gewichtet wird als die wachsenden, mit der bewegten Blende geöffneten Teilfelder. Der größte Wichtungsfaktor für ein Teilfeld beträgt bei üblichen Isodosenverläufen nur 4% des Wichtungsfaktors für das vollständige offene Feld. Die analytische Vorgabe der Blendenbewegung ist rechnerisch wesentlich schneller durchzuführen als ein iteratives Verfahren.

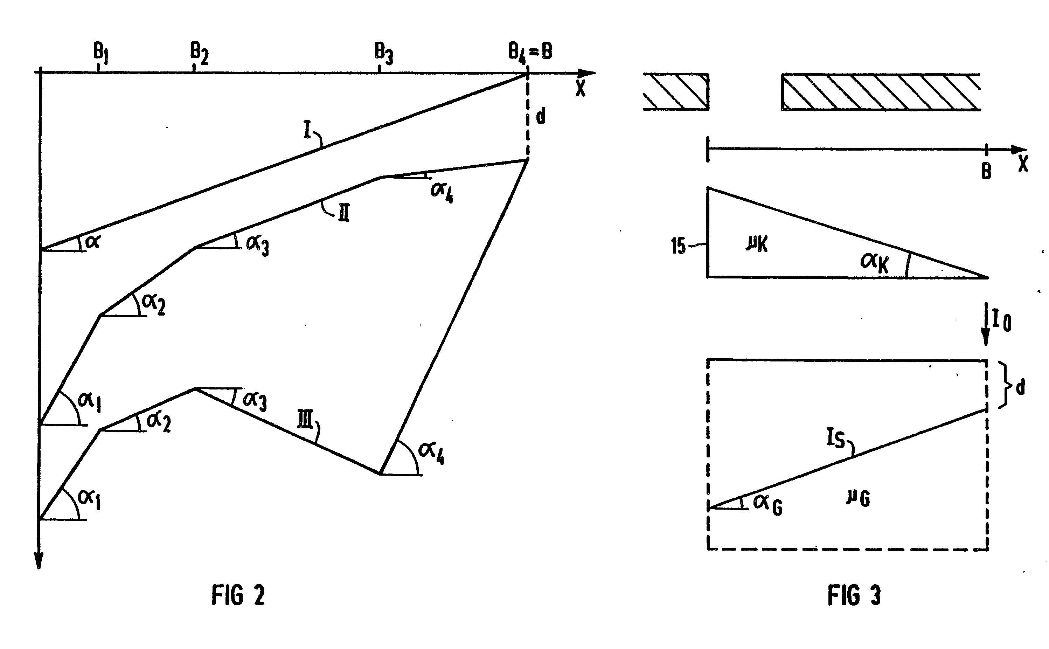

In Fig. 2 sind verschiedene Isodosenverläufe dargestellt. Wenn man davon ausgeht, daß sich die Blende 12a vom geschlossenen Zustand ($x = O$) zum geöffneten Zustand ($x = B$) bewegt, so können damit nur Isodosen des in Fig. 2 mit I bzw. II bezeichneten Typs generiert werden, d.h., Isodosen mit $\tan\alpha > 0$. Es gilt nämlich:

$I(x) = I_0 \exp(-\mu \ x \ tg \ \alpha)$,

da $I(x) < I_0$ und $x, \mu > 0$

folgt $tg \ \alpha \geq 0$ und $O° \leq \alpha \leq 90°$.

Damit ist eine Einschränkung des einstellbaren Isodosenverlaufes verbunden, die mit der folgenden, anhand von Fig. 3 erläuterten Anordnung überwunden werden soll. Dabei wird dem System Blendenanordnung 12-Bestrahlungsobjekt die Wirkung eines realen Keilfilters 15 mit einem festen Winkel $\alpha_K$ und dem Schwächungskoeffizienten $\mu_K$ überlagert. In diesem Fall kann $\alpha$ auch negativ werden. Das Filter kann im Prinzip jede beliebige Form haben; es muß lediglich die Bedingung erfüllt sein, daß die Strahlenabsorption mit zunehmendem x-Wert (d.h. in Öffnungsrichtung der Blende 12a) abnimmt. Im folgenden wird die Konfiguration für einen keilförmigen Filter mit einem Keilwinkel $\alpha_k$ betrachtet. Für den Dosiswert $I(x)$ gilt:

$I(x) = I_s \exp\{\mu_G [d + (B-x) \ tg\alpha_G] \cdot \exp[\mu_K (B-x) \ tg\alpha_K]\}$

Dabei ist $I_s$ der Dosiswert der Isodose, die unter dem Winkel $\alpha_G$ in der Tiefe d verlaufen soll. Der Index G steht hier für Gewebe.

Für die Freiluftdosis $I_0$ in Höhe der Körperoberfläche (ohne Wirkung von Blende 12a und Filter 15) gilt:

$I_0 = I_s \exp[\mu_G (d + B \ tg\alpha_G) + \mu_K B \ tg\alpha_K)]$

Damit folgt:

$I(x) = I_0 \exp[-x (\mu_G \ tg\alpha_G + \mu_K \ tg\alpha_K)]$

Analog zu den oben angeführten Betrachtungen muß gelten:

$\mu_G \ tg\alpha_G = \mu_K \ tg\alpha_K \geq 0$

Somit folgt:

$$tg \ \alpha_G \geq - \frac{\mu_K}{\mu_G} \ tg \, \alpha_K$$

Damit ist der Bereich der möglichen Isodosenwinkel $\alpha_G$ definiert, wobei man erkennt, daß sowohl positive als auch negative Werte möglich sind.

Für den Fall, daß die Isodose nur einen konstanten Winkel $\alpha_G$ besitzen soll, kann man folgende Bewegungsgleichung ableiten:

$$x(t) = \frac{\ln \frac{k}{I_0} \ (T + tc - t)}{\mu_G \ tg \, \alpha_G + \mu_K \ tg \, \alpha_G}$$

Die für eine Isodose mit konstantem Winkel $\alpha_G$ angegebene Bewegungsgleichung läßt sich auch für den Fall verallgemeinern, daß die Isodose für die Koordinate x unterschiedliche Winkelsegmente $\alpha_i$ aufweist. Für den Bereich i gilt folgende Bewegungsgleichung:

$$x_i(t) = \frac{\ln \frac{k}{I_0} (T+tc-t) + \mu \sum_{i=1}^{i} B_{i-1} (tg\,\alpha_{i-1} - tg\,\alpha_i)}{\mu\,tg\,\alpha_i + \mu_K\,tg\,\alpha_K}$$

$$v_i(x) = \frac{k}{I_0} \frac{\exp\left[(x-B_{i-1})(\mu\,tg\,\alpha_i+\mu_K\,tg\,\alpha_K)+\sum_{i=2}^{i-1}(B_{i-1}-B_{i-2})(\mu\,tg\,\alpha_i+\mu_K\,tg\,\alpha_K)\right]}{\mu\,tg\,\alpha_i + \mu_K\,tg\,\alpha_K}$$

Für den Fall, daß man nur in x-Richtung ansteigende Isodosen erzeugen will, kann man den realen Keilfilter 15 weglassen und die obengenannte Gleichung vereinfacht sich mit tg $\alpha_K = 0$ und $\mu_K = 0$.

Die beschriebene Anwendung einer Rechner-gesteuerten Blende bei gleichzeitigem Einsatz eines realen Keilfilters 3 ermöglicht eine große Variation bei der Festlegung des Isodosenverlaufes, wobei auch in Öffnungsrichtung der Blende zunehmende Dosisleistungen erzielt werden können. Durch bewegte Blenden allein könnte ein solcher Dosisverlauf überhaupt nicht erzielt werden. Eine Bestimmung komplexerer Isodosenverläufe allein durch Filter wäre zwar im Prinzip möglich; jedoch aufgrund der Vielzahl von speziellen Filterformen praktisch nicht sinnvoll. Durch die dargestellte Kombination von bewegter Blende und realem Keilfilter dagegen erhält man bereits mit einem einzigen Keilfilter eine große Variationsbreite bei der Festlegung der Isodosen, auch bei mehreren unterschiedlichen Winkelsegmenten. Selbst bei komplizierten Isodosenverläufen läßt sich die Bewegungsgleichung für die Blende rein analytisch angeben, d.h. aufwendige iterative Rechenverfahren sind nicht erforderlich.

**Ansprüche**

1. Strahlentherapiegerät mit einer Strahlenquelle (2) und einer zwischen Strahlenquelle (2) und Bestrahlungsobjekt angeordneten, ein Bestrahlungsfeld bestimmenden Blendenanordnung (12), wobei während der Bestrahlung mindestens eine Blende (12a) der Blendenanordnung (12) durch eine Steuereinrichtung (13) so bewegbar ist, daß eine in Öffnungsrichtung der Blende (12a) abfallende Dosisleistung im Bestrahlungsfeld erzielbar ist, **dadurch gekennzeichnet,** daß zusätzlich ein nicht beweglicher Filterkörper (15) in den Strahlengang einfügbar ist, der eine in Öffnungsrichtung der Blende (12a) abnehmende Absorption aufweist.

2. Strahlentherapiegerät nach Anspruch 1, **dadurch gekennzeichnet,** daß die Strahlenquelle (2) aus einem Elektronenbeschleuniger (2a) mit einem Umlenkmagneten (2b) besteht.

3. Strahlentherapiegerät nach Anspruch 1 oder 2, **dadurch gekennzeichnet,** daß der Filterkörper (15) eine Keilform aufweist.

4. Strahlentherapiegerät nach Anspruch 1, **dadurch gekennzeichnet,** daß der mit der bewegten Blende (12a) erzeugte Isodosenwinkel für verschiedene Bereiche des Bestrahlungsfeldes unterschiedlich einstellbar ist.

5. Strahlentherapiegerät nach einem der Ansprüche 1 bis 4, **dadurch gekennzeichnet,** daß die Bewegung der Blende (12a) nach folgender Gleichung analytisch vorgegeben wird:

$$x_i(t) = \frac{\ln \frac{k}{I_0} (T+tc-t) + \mu \sum_{i=1}^{i} B_{i-1} (tg\,\alpha_{i-1} - tg\,\alpha_i)}{\mu\,tg\,\alpha_i + \mu_K\,tg\,\alpha_K}$$

Dabei gilt:

x = x-Koordinate der Blende 12a

K = Dosisleistung in Luft bei der Tiefe O

$I_0$ = Dosis in Luft bei der Tiefe O

T = Zeit, in der sich die Blende (12a) vom geschossenen Zustand in den geöffneten Zustand bewegt

$t_c$ = Zeitanteil, in dem die Bestrahlung bei voll geöffneter Blende (12a) weitergeführt wird

$\mu$ = effektiver linearer Schwächungskoeffizient

$B_i$ = x-Koordinate am Ende eines Winkelsegmentes

$\alpha_i$ = Isodosenwinkel im jeweiligen Segment

$\alpha_K$ = Winkel des realen Filterkörpers

$\mu_K$ = Schwächungskoeffizient des realen Winkelkörpers

Der Index i kennzeichnet ein Segment mit konstantem Isodosenwinkel $\alpha_i$.

FIG 1

**FIG 3**

**FIG 2**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-3 704 795 (PHILIPS)<br>* Spalte 3, Zeile 50 - Spalte 4, Zeile 17 *<br>--- | 1,3,4 | A 61 N 5/10 |
| A | ELECTROMEDICA, vol. 36, no. 3, 3/1968, Seiten 67-69; A. VON SONNTAG et al.: "Keilfilter in der Co-60-Teletherapie"<br>--- | 1-4 | |
| A | MEDICAL PHYSICS, vol. 9, no. 2, März-April 1982, Seiten 204-207, Am. Assoc. Phys. Med., New York, US; R.K. TEN HAKEN et al.: "The use of nonhydrogenous wedges for therapeutic neutron beam shaping"<br>--- | 1-4 | |
| A,D | MEDICAL PHYSICS, vol. 5, no. 5, September/Oktober 1978, Seiten 426-429, Am. Assoc. Phys. Med.; P.K. KIJEWSKI et al.: "Wedge-shaped dose distributions by computer-controlled collimator motion"<br>----- | 1,5 | RECHERCHIERTE SACHGEBIETE (Int. Cl.5)<br><br>A 61 N<br>G 21 K |

Der vorliegende Recherchenbericht wurde für alle Patentansprüche erstellt

| Recherchenort | Abschlußdatum der Recherche | Prüfer |
|---|---|---|
| DEN HAAG | 12-12-1989 | LEMERCIER D.L.L. |